# EUROPEAN PATENT APPLICATION

(11) **EP 3 974 014 A1**
(43) Date of publication of application: **30.03.2022**
(21) Application number: 21000266.3
(22) Date of filing: 22.09.2021
(51) Int. Cl.: A61M 15/00

(54) **INHALER FOR DELIVERING MEDICINES**

(30) Priority: 24.09.2020 PL 43543520
(71) Applicant: Polfarmex S.A., 99-300 Kutno (PL)
(72) Inventor: Prus, Artur, Warsaw (PL)

(57) **Abstract**

An inhaler for administering a medicament in the form of dry powder from blister packs seated on a conveyor, wherein two geometrically identical blister packs (01) wound on the conveyor (06) are moved simultaneously by a step determined by the distance between the pockets (01.1) with the medicament in each blister strip (01), and the pockets are positioned centrally to the axes of the air openings (05.1) of the body (05), the blister packs with the medicament (01) being placed in the chambers (05.2) of the body (05), whereas one of the torn ends of the blister pack (01.2.1) of the lidding foil (01.2) is attached to the slit (12.1) of a pulling roller (12), and the other end (01.3.1) of the pocket foil (01.3) to the pin (09.1) of a rotor hook (09), the lidding layer (01.2) being wound onto the rotor (11) by a constant value, adjusted by flexible wings (11.1) and the gear ratio of gear wheels, the rotor (11) being seated on the rotor hook (09), which is seated on a rotor wheel (10), the pulling roller (12) and the rotor gear wheel (10) along with the seated hook (09) and rotor (11) being coupled with the conveyor (06) by means of intermediate wheels (13) separately for each blister pack set, the conveyors (06) rotating to dispense the medicament, driven by a series of gear wheels (16), (17), (18) and a rack (19) due to the movement of a slide (04).

## Description

The object of the invention is an inhaler allowing for the dispensing of a medicament from a blister pack by inhalation.

In known solutions, blister packs are wound into a loose roll and placed in a chamber. Their movement is caused by the rotations of a rotor and a conveyor. The layer of blister pack pockets is further wound onto a roller and stored in a separate chamber.

The European patent with the publication number EP 2432530 describes an inhaler for delivering a dry-powder medicament to a patient from an open blister pocket of a blister pack, the blister pack comprising a plurality of blister pockets spaced apart in the length direction of the pack, each of them containing a measured dose of the medicament, the inhaler comprising: a housing enclosing used and unused portions of the blister pack, and a medicament dispensing mechanism for opening the blister pockets of the blister pack; and a manifold through which air can be drawn in during use, the manifold comprising an air inlet for receiving external air, at least one medicament aperture for communicating with the open pocket of the blister pack to enable entrainment of the medicament by the air drawn through the manifold, and an air outlet for the delivery of the entrained medicament to the patient, the inhaler being characterised in that the manifold and a part of the housing are provided as a unitary moulded plastics component, and wherein the unitary component defines, in use, at least a portion of the outer surface of the inhaler, in which outer surface the air inlet is arranged.

The inhaler disclosed in US patent no. 5,590,645 comprises a medicament-dispensing mechanism, which provides peeling parts of a lid sheet off a base sheet of a blister pack, due to which a blister pocket is opened during every activation of the inhaler. This mechanism comprises means for sliding the blister pack in order to move an open pocket to a position aligned with the medicament openings made in the manifold of the inhaler, the manifold being connected in a manner enabling the flow of fluid to a mouthpiece, by means of which the patient can perform the inhalation. The unused portion of the blister pack and the used portions of the base sheet and the lid sheet are stored inside the body of the inhaler. In use of the inhaler described above, the patient exposes the mouthpiece by rotating the cover relative to the body of the inhaler, upon which they use an activating element to activate the medicament-dispensing mechanism. Subsequently, the patient performs inhalation by means of the mouthpiece, drawing air from inside the body of the inhaler through the manifold. The stream of air flowing through the mouthpiece and the manifold entrains the medicament in the form of dry powder placed in the open blister pocket, this medicament being delivered to the patient in the form of an aerosol. Subsequently, the activating element and the cover of the inhaler are placed in their initial positions, which is intended to prepare the medicament-dispensing mechanism for another use.

In the patent description published with the number PL 207319, a powder inhaler comprises a container storing a powdered medicament, a metering member having a dosing recess, and a mouthpiece connected to an inhalation channel, enabling inhalation of a dose of the powdered medicament contained in the dosing recess of the metering member, when the metering member is in an inhalation position, the metering member being slidingly situated between a filling position, in which the dosing recess is situated opposite the opening in the container and it becomes filled with a dose of the powdered medicament, and the inhalation position, in which the dosing recess is situated opposite the inhalation channel, a protective member being situated between the metering member and the inhalation channel, slideable between a closed position, in which the protective member covers at least the dosing recess of the metering member, when the metering member is in the inhalation position, therefore preventing the powdered medicament contained in the dosing recess from entering the inhalation channel, and an open position, in which the protective member does not cover the dosing recess, exposing the dosing recess from the side of the inhalation channel, enabling the inhalation of the dose of the powdered medicament contained in the dosing recess.

Although inhalers for dry powder known from prior art provide improved invariability of the size of doses administered to a patient, there is still a need for inhalers providing improved invariability of the dose size and safety of administering the medicament. At the same time, there is a need for inhalers allowing for minimising the risk of accidental exposure of the mouthpiece. In addition, a common problem in dry powder inhalers with tearable blister packs is the smoothness of operation and the rather considerable force needed to activate the mechanism.

The essence of the invention is an inhaler for administering a medicament in the form of dry powder, characterised by simultaneous movement of two geometrically identical blister packs wound on a conveyor by a step determined by the distance between the pockets with the medicament in each blister strip, and by positioning the pockets centrally to the axes of air openings of the body, the blister packs with the medicament being placed in chambers of the body, whereas one of the torn ends of the blister pack, the lidding foil, is attached to the slit of a pulling roller, and the other end of the pocket foil to the pin of a rotor hook, the lidding layer being wound onto the rotor by a constant value, adjusted by flexible wings and the gear ratio of gear wheels, the rotor being seated on the rotor hook, which is seated on a rotor wheel, the pulling roller and the rotor gear wheel along with the seated hook and the rotor being coupled with the conveyor by means of intermediate wheels separately for each blister pack set, the conveyors rotating to dispense the medicament, driven by a series of gear wheels and a rack due to the movement of a slide.

Preferably, the individual parts of the mechanism are seated on a lower housing and covered by a body forming chambers for the coils of the blister pack: a lidding layer chamber, a base layer chamber and a chamber for an untorn blister pack, the whole being enclosed by an upper housing.

Preferably, in order to prevent accidental/unauthorised opening of the slide, an elastic locking element is introduced, wherein, in order to activate the dosing mechanism, it is necessary to press a lever along with simultaneous movement of the slide; the slide is to be moved to a stop on the upper housing having pre-set values of movement, enabling the rotation of the conveyors by the value of graduation of the pockets of the blister packs and positioning of the open pockets opposite the air channels, the movable slide being connected by hooks to hook slots of the rack, the rack being coupled with the gear wheels, the gear wheels being coupled with the gear wheels of the catchers of the conveyor, which via the wings of the latch rotate the conveyors along with the blister packs wound thereon. Preferably, the inhaler is provided with a dose counter in the form of a disc with consecutively printed dose numbers, the dose counter being coupled with the pulling roller of one of the blister packs.

Preferably, the reading of the number of doses remaining for the patient is placed on the same wall as the mouthpiece, making it easier to check the number of inhalations possible to perform through the window of the dose counter placed in the lower housing.

Preferably, a high gear ratio is used on the gear wheels, causing the force necessary to activate the mechanism to be minimised.

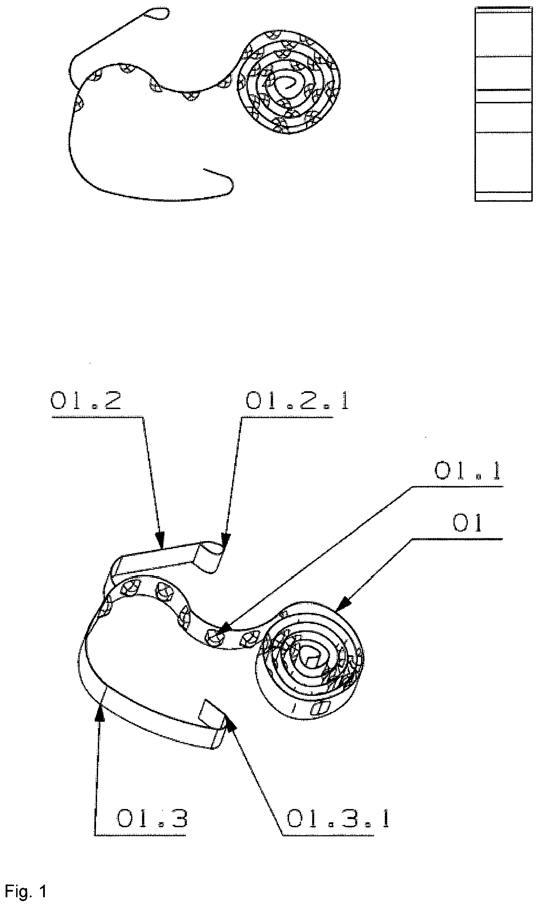

Fig. 1 is a general view of the blister pack showing the key elements.

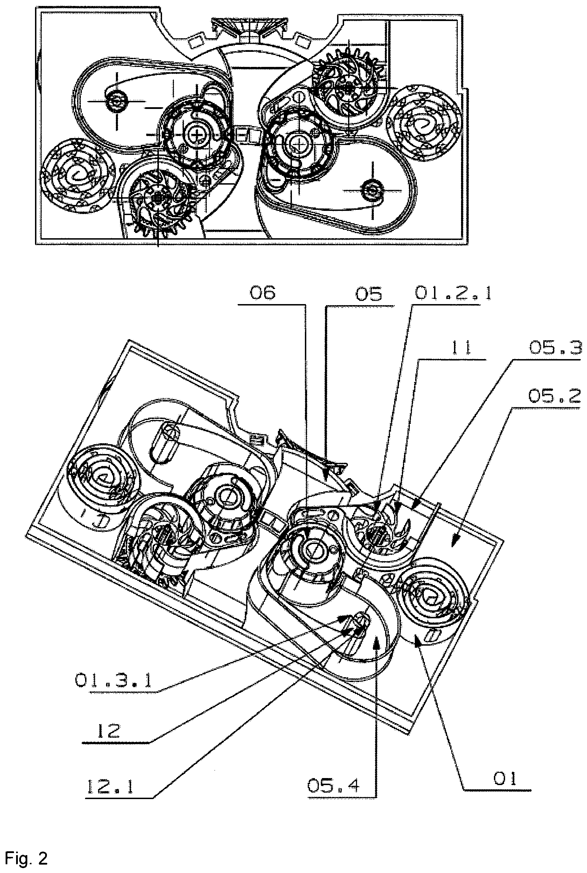

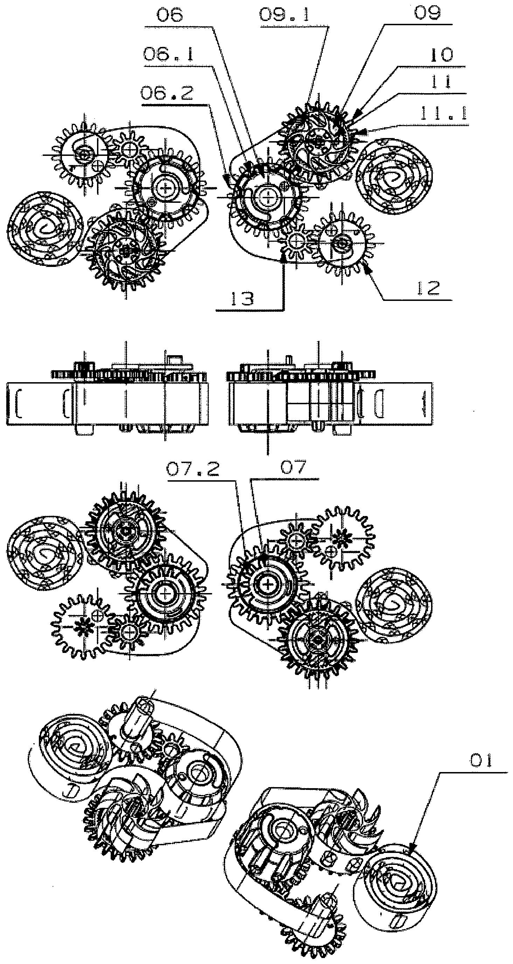

Fig. 2 is a general view of the placement of the blister packs in the housing and the body.

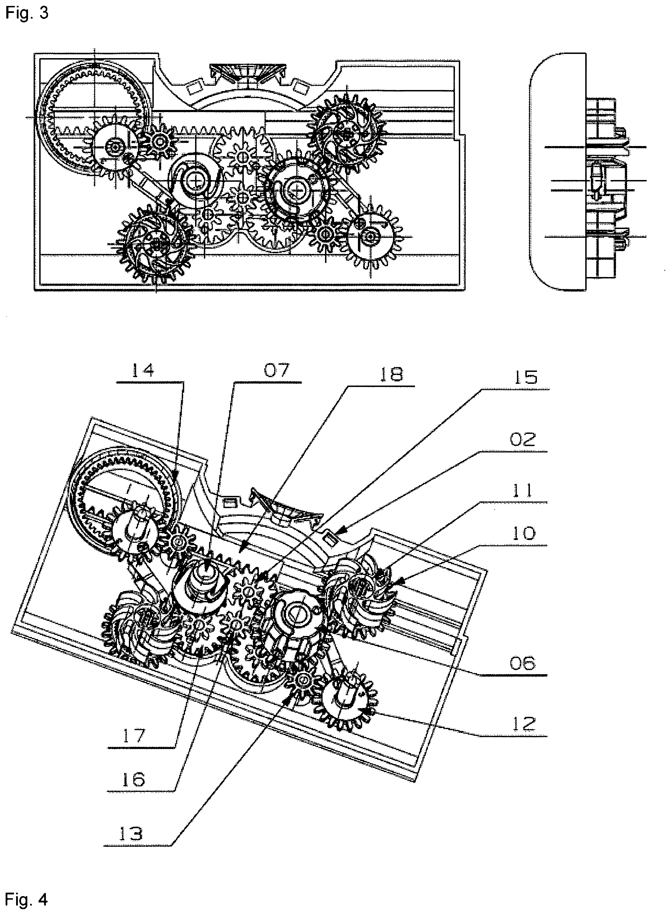

Fig. 3 is a general view of the manner of mounting the blister pack on the transport elements, also showing the key elements.
Fig. 4 is a general view of the arrangement of gear wheels in the housing.

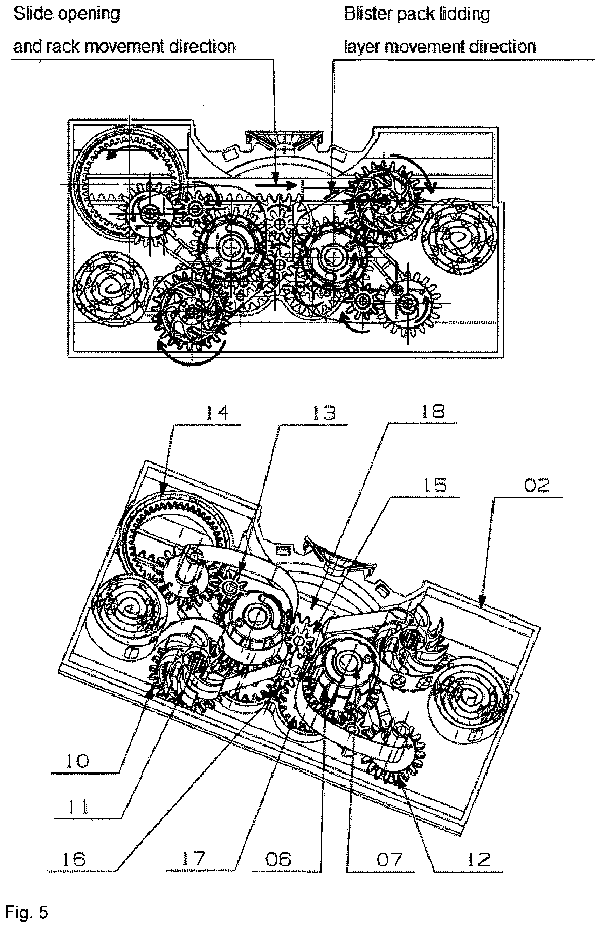

Fig. 5 presents the operating principles of the mechanism, the direction of rotations for the gear wheels of the mechanism and of movement for the layer of blister packs.

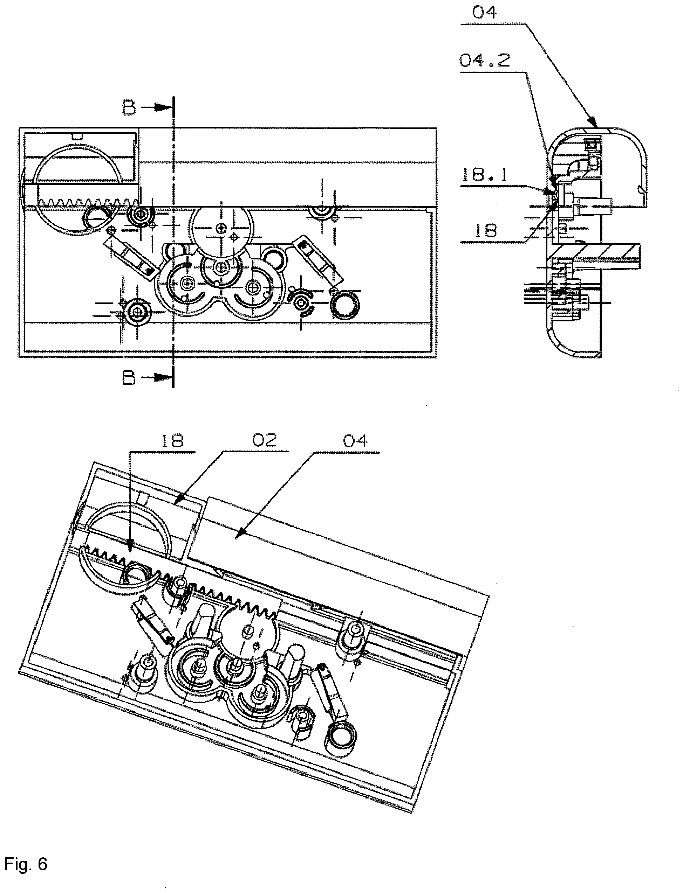

Fig. 6 is a general view of the connection between the rack and the slide.

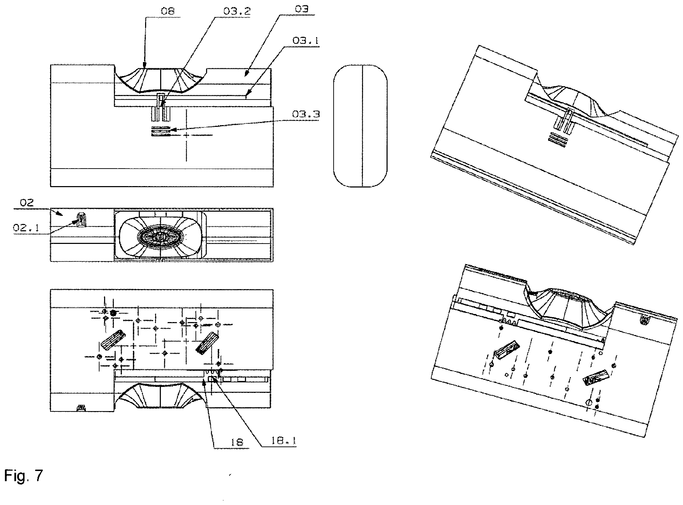

Fig. 7 presents the housings with the mouthpiece.

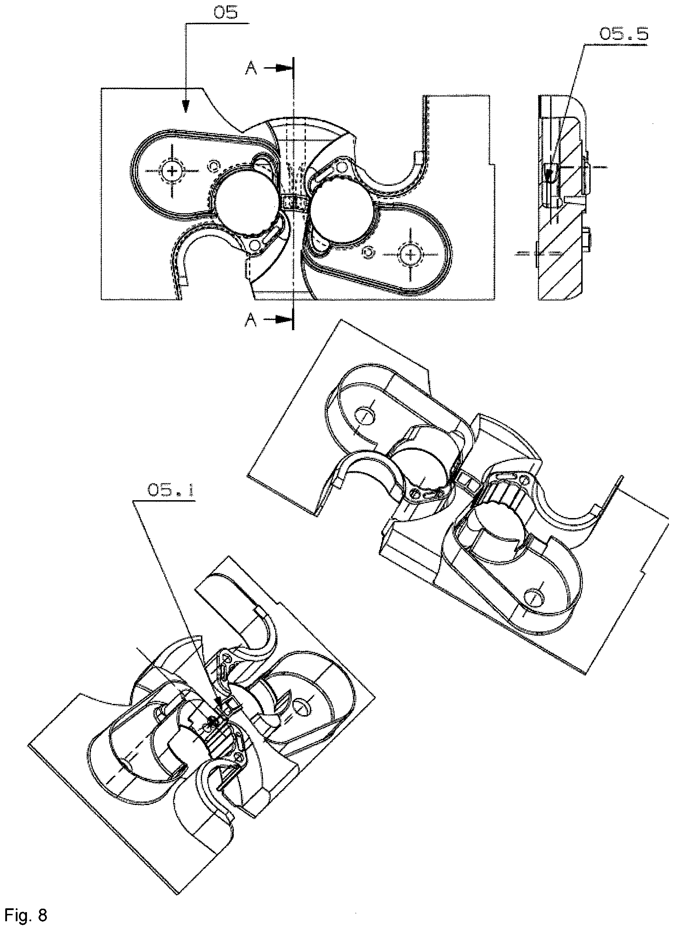

Fig. 8 presents the body, the air channels.

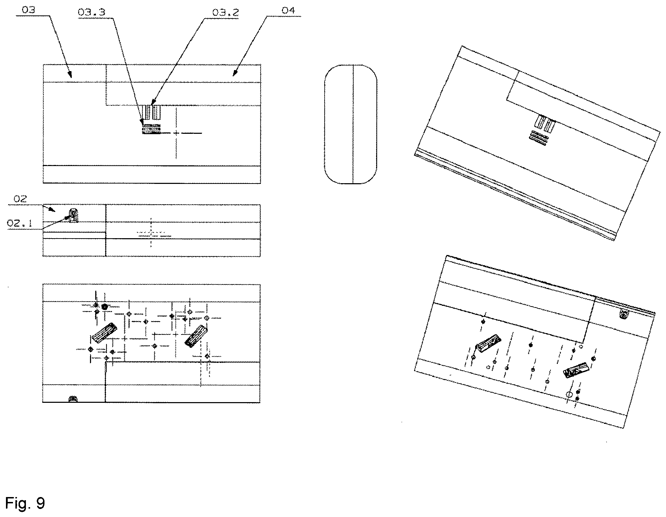

Fig. 9 presents an overall view of the entire inhaler in a position with the slide closed, also showing the key elements.

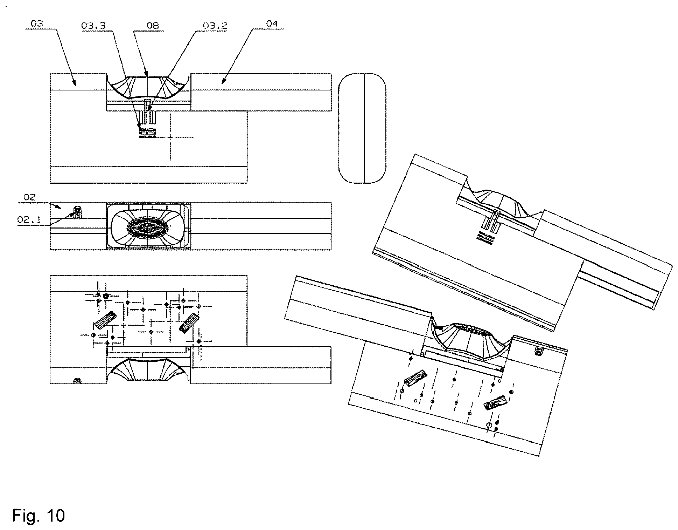

Fig. 10 presents an overall view of the entire inhaler in a position with the slide opened, also showing the key elements.

List of parts:
01 Blister pack
   01.1 blister pack pocket
   01.2 lidding layer
      01.2.1 lidding layer end
   01.3 layer of pockets
      01.3.1 end of the layer of pockets
02 lower housing
   02.1 dose window
03 upper housing
   03.1 stop
   03.2 slide locking lever
   03.3 air channels
04 slide
   04.1 locking lever hook
   04.2 rack hooks
   04.3 upper housing hooks
05 body
   05.1 air channels
   05.2 blister pack chamber
   05.3 blister pack lidding layer chamber
   05.4 base layer chamber
   05.5 mixing chamber
06 conveyor
   06.1 blister pack pocket
   06.2 gear wheel
   06.3 conveyor catcher latch slot
07 conveyor catcher
   07.1 latch wings
   07.2 gear wheel
08 mouthpiece
   08.1 air channel
09 rotor hook
   09.1 pin
10 rotor wheel
11 rotor
   11.1 rotor wings
12 pulling roller
   12.1 mounting slit
13 intermediate wheel
14. dose counter
15. gear wheel 1
16. gear wheel 2
17. gear wheel 3
18. rack
   18.1 rack hook slots

According to the present embodiment, the operation of the inhaler involves simultaneous movement of two geometrically identical blister packs 01 by a specified step determined by the distance between the pockets 01.1 with the medicament in each blister pack strip 01 and the positioning of the pockets centrally to the axes of air openings 05.1 of the body 05. Blister packs with medicaments 01 are placed in the chambers 05.2 of the body 05 of the device. One of the torn ends of the blister pack 01.2.1 - the lidding foil 01.2 - is attached to the slit 12.1 of the pulling roller 12. The other end 01.3.1 - the pocket foil 01.3 - to the pin 09.1 of the rotor hook 09. The lidding layer 01.2 is wound onto the rotor 11 by a constant value, which is adjusted by flexible wings 11.1 and the selected gear ratio of the gear wheels of the mechanism. The rotor 11 is seated on the rotor hook 09, which is seated on a rotor wheel 10. The blister pack is wound on the conveyor 06. The pulling roller 12 and the rotor gear wheel 10 along with the seated hook 09 and rotor 11 are coupled with the conveyor 06 by means of intermediate wheels 13, separately for each blister pack set. The conveyors 06 rotate by a constant angle resulting from the distance between the pockets 01.1 on the blister packs 01, and they position the exposed pockets 01.1 centrally to the air flow openings 05.1. The conveyors 06 rotate to dose the medicament, driven by a series of gear wheels 16, 17, 18 and a rack 19 due to the movement of the slide 04.

The individual parts of the mechanism are seated on the lower housing 02 and covered by the body 05 forming chambers for the coils of the blister pack: a lidding layer chamber 05.3, a base layer chamber 05.4 and a chamber 05.2 for an untorn blister pack 01. The whole is enclosed by an upper housing 03. The described embodiment uses a mechanism converting the linear motion of the slide 04 to the rotary motion of the conveyors 06. The movable slide 04 serves two functions: 1 - it protects the mouthpiece 08 against dirt and 2 - it serves as a lever for activating the dosing mechanism. In order to prevent accidental/unauthorised opening of the slide 04, which could result in releasing a dose of the medicament, in the solution, an elastic locking element 03.2 has been introduced in the upper housing 03. In order to activate the dosing mechanism, it is necessary to press the lever 03.2 along with simultaneous movement of the slide 04. The slide 04 is to be moved to the stop 03.1 on the upper housing 03, with a pre-set value of movement allowing for rotation of the conveyors 06 by the value of graduation of the pockets of the blister packs 01 and positioning the open pockets 01.1 opposite the air channels 05.1. The movable slide 04 is connected by hooks 04.2 to the hook slots 19.1 of the rack 19. The rack 19 is coupled with the gear wheels 16, 17 and 18. The gear wheels 18 are coupled with the gear wheels 07.2 of the catchers of the conveyor 07, which via the wings of the latch 07.1 rotate the conveyors (06) along with the blister packs 01 wound thereon. Since one end of the blister pack 01 is separated and one of the separated ends 01.3.1 is attached to the pulling roller 12 and the other one to the rotor 11, during rotation of the conveyors 06 and movement of the blister pack 01, it becomes further torn, in order to expose a pocket with the medicament positioned opposite the air channel. In order to gain access to the healing content of the open pockets in the blister packs, the patient breathes in, with their mouth tightly contacting the mouthpiece 08. This results in sucking the air into the distribution channels 05.1, and in flow through the open pockets of the blister packs 01.1.

This causes the powdered medicament present in the open pockets to transform into an aerosol and flow into the mixing chamber 05.5, where it mixes with itself, and then to the outlet of the mouthpiece 08. The mixing of powder takes place in the mixing chamber 05.5 during the transport of powder from each open pocket. Upon administering the medicament, the patient covers the mouthpiece 08 with the slide 04. Because of the latch wings 07.1 in the latch slot 06.3 and the latch of the lower housing 02.4, the rotation of the rotors 11 and conveyors 06 is blocked, and it is only possible to cover the mouthpiece 08, without setting the entire mechanism in a return motion. In this solution, the device is provided with a dose counter in the form of a disc 14 with consecutively printed dose numbers. The dose counter is coupled with the pulling roller 12 of one of the blister packs. In this solution, the reading of the number of doses remaining for the patient is placed on the same wall as the mouthpiece 8, making it easier to check the number of inhalations possible to perform through the window of the dose counter 02.3 placed in the lower housing 02.

The inhaler according to the invention provides invariability of the dose size, also minimising the risks of accidental exposure of the mouthpiece, and results in a need to use minor force in order to activate the mechanism, ensuring smoothness of operation.

## Claims

1. An inhaler for administering a medicament in the form of dry powder from blister packs seated on a conveyor, the lidding layer of the blister pack being torn before a mixing chamber, whereas the medicament in the form of powder is transported by inhalation of the patient's mouth touching the mouthpiece, resulting in suction and flow through an open blister pack pocket, **characterised in that** two geometrically identical blister packs (01) wound on the conveyor (06) are moved simultaneously by a step determined by the distance between the pockets (01.1) with the medicament in each blister strip (01), and the pockets are positioned centrally to the axes of air openings (05.1) of the body (05), the blister packs with the medicament (01) being placed in the chambers (05.2) of the body (05), whereas one of the torn ends of the blister pack (01.2.1) of the lidding foil (01.2) is attached to the slit (12.1) of a pulling roller (12), and the other end (01.3.1) of the pocket foil (01.3) to the pin (09.1) of a rotor hook (09), the lidding layer (01.2) being wound onto the rotor (11) by a constant value, adjusted by flexible wings (11.1) and the gear ratio of gear wheels, the rotor (11) being seated on the rotor hook (09), which is seated on a rotor wheel (10), the pulling roller (12) and the rotor gear wheel (10) along with the seated hook (09) and rotor (11) being coupled with the conveyor (06) by means of intermediate wheels (13) separately for each blister pack set, the conveyors (06) rotating to dispense the medicament, driven by a series of gear wheels (16, 17, 18) and a rack (19) due to the movement of a slide (04).

2. The inhaler for administering a medicament in the form of dry powder according to claim 1, **characterised in that** individual parts of the mechanism are seated on a lower housing (02) and covered by a body (05) forming chambers for the coils of the blister pack: a lidding layer chamber (05.3), a base layer chamber (05.4) and a chamber (05.2) for an untorn blister pack 01, the whole being enclosed by an upper housing (03) .

3. The inhaler for administering a medicament in the form of dry powder according to claim 2, **characterised in that**, in order to prevent accidental/unauthorised opening of the slide (04), an elastic locking element (03.2) is introduced, wherein, in order to activate the dosing mechanism, it is necessary to press a lever (03.2) along with simultaneous movement of the slide (04); the slide (04) is to be moved to a stop (03.1) on the upper housing (03) having pre-set values of movement, enabling the rotation of the conveyors 06 by the value of graduation of the pockets of the blister packs 01 and positioning the open pockets (01.1) opposite the air channels (05.1), the movable slide (04) being connected by hooks (04.2) to hook slots (19.1) of a rack (19), the rack (19) being coupled with gear wheels (16, 17) and (18), the gear wheels (18) being coupled with the gear wheels (07.2) of the catchers of the conveyor (07), which via the wings of the latch (07.1) rotate the conveyors (06) along with the blister packs (01) wound thereon.

4. The inhaler for administering a medicament in the form of dry powder according to claim 1, **characterised in that** it is provided with a dose counter in the form of a disc (14) with consecutively printed dose numbers, the dose counter being coupled with the pulling roller (12) of one of the blister packs.

5. The inhaler for administering a medicament in the form of dry powder according to claim 4, **characterised in that** the reading of the number of doses remaining for the patient is placed on the same wall as the mouthpiece (8), making it easier to check the number of inhalations possible to perform through the window of the dose counter (02.3) placed in the lower housing (02).

6. The inhaler for administering a medicament in the form of dry powder according to any of the preceding claims, **characterised in that** it uses a high gear ratio on the gear wheels, causing the force needed to activate the mechanism to be minimised.
